(19) Europäisches Patentamt
European Patent Office
Office européen des brevets

(11) **EP 3 354 239 B1**

(12) **EUROPÄISCHE PATENTSCHRIFT**

(45) Veröffentlichungstag und Bekanntmachung des Hinweises auf die Patenterteilung:
**24.08.2022   Patentblatt 2022/34**

(21) Anmeldenummer: **18156076.4**

(22) Anmeldetag: **22.10.2012**

(51) Internationale Patentklassifikation (IPC):
**A61F 9/008** (2006.01)

(52) Gemeinsame Patentklassifikation (CPC):
**A61F 9/00825;** A61F 2009/0087; A61F 2009/00889; A61F 2009/00897

(54) **ERZEUGUNG VON SCHNITTFLÄCHEN IN EINEM TRANSPARENTEN MATERIAL MITTELS OPTISCHER STRAHLUNG**

PRODUCING CUT SURFACES IN A TRANSPARENT MATERIAL BY MEANS OF OPTICAL RADIATION

PRODUCTION DE SURFACES DE COUPE DANS UN MATÉRIAU TRANSPARENT À L'AIDE D'UN RAYONNEMENT OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.10.2011   DE 102011085046**

(43) Veröffentlichungstag der Anmeldung:
**01.08.2018   Patentblatt 2018/31**

(62) Dokumentnummer(n) der früheren Anmeldung(en) nach Art. 76 EPÜ:
**12781061.2 / 2 768 447**

(73) Patentinhaber: **Carl Zeiss Meditec AG**
**07745 Jena (DE)**

(72) Erfinder:
• **PAPASTATHOPOULOS, Evangelos**
**71638 Ludwigsburg (DE)**
• **STOBRAWA, Gregor**
**07743 Jena (DE)**
• **BISCHOFF, Mark**
**07749 Jena (DE)**

(74) Vertreter: **Patentanwälte Geyer, Fehners & Partner mbB**
**Perhamerstrasse 31**
**80687 München (DE)**

(56) Entgegenhaltungen:
EP-A1- 1 486 185         WO-A2-2006/074469
DE-A1-102009 012 873     US-A1- 2009 171 329
US-A1- 2011 022 036

**Beschreibung**

Die Erfindung bezieht sich auf ein Verfahren zur Erzeugung von Steuerdaten für eine Erzeugung einer Schnittfläche in einem transparenten Material, wobei die Steuerdaten für eine Lasereinrichtung ausgebildet sind, die dazu ausgebildet ist, mittels optischer Strahlung eine Trennung innerhalb des transparenten Materials zu bewirken, und die aufweist eine Optik, die optische Strahlung längs einer optischen Achse in einen im Material gelegenen Fokus bündelt und im Material ein Bildfeld hat, in dem der Fokus liegt und das eine Bildfeldgröße hat, eine Einrichtung zur Verstellung einer Lage des Fokus quer zur optischen Achse und längs der optischen Achse, die Steuerdaten eine Bahnkurve für eine Verstellung der Lage des Fokus der optischen Strahlung im Material zur Erzeugung der Schnittfläche derart vorgeben, dass die Schnittfläche sich parallel zur optischen Achse erstreckt, in Projektion längs der optischen Achse eine Kurve ist und in Projektion längs der optischen Achse eine Maximalausdehnung aufweist.

Die Erfindung bezieht sich weiter auf eine Bearbeitungsvorrichtung zur Erzeugung einer Schnittfläche in einem transparenten Material, die umfasst eine Lasereinrichtung, die dazu ausgebildet ist, mittels optischer Strahlung eine Trennung innerhalb des transparenten Materials zu bewirken, und die aufweist eine Optik, die optische Strahlung längs einer optischen Achse in einen im Material gelegenen Fokus bündelt und im Material ein Bildfeld hat, in dem der Fokus liegt und das eine Bildfeldgröße hat, eine Einrichtung zur Verstellung einer Lage des Fokus quer zur optischen Achse und längs der optischen Achse, eine Steuereinrichtung, welche mit der Lasereinrichtung verbunden ist und die Lasereinrichtung so ansteuert, dass deren Einrichtung zur Verstellung der Lage des Fokus die Lage des Fokus der optischen Strahlung im Material entlang einer Bahnkurve verschiebt, wobei die Steuereinrichtung die Lasereinrichtung so ansteuert, dass die Schnittfläche sich parallel zur optischen Achse erstreckt, in Projektion längs der optischen Achse eine Kurve ist und in Projektion längs der optischen Achse eine Maximalausdehnung aufweist.

Diese Verfahren bzw. Vorrichtung werden insbesondere im Gebiet der Ophthalmologie verwendet, und sind z. B. aus der US 2011/0022036 A1 WO2006/074469 und US2009/171329 bekannt.

Bei diesen Anwendungsgebieten, wie auch bei anderen Applikationen, wirkt die optische Strahlung im Inneren des Materials, beispielsweise des Gewebes, das für die optische Strahlung transparent ist. Üblicherweise werden nichtlineare Prozesse eingesetzt, welche eine Fokussierung von Bearbeitungsstrahlung, üblicherweise gepulste Laserstrahlung, in das Material hinein, d. h. unter die Oberfläche des Materials, benötigen. Die Erzeugung einer Schnittfläche geschieht dadurch, dass die Lage des Fokus im Material verschoben wird. In dem Verständnis, das dieser Beschreibung zu Grunde liegt, bedingt die Verschiebung des Fokus nicht zwingend, dass zu diesem Zeitpunkt auch Strahlung in den Fokus abgegeben wird. Insbesondere beim Einsatz gepulster Laserstrahlung wird der Fokus kontinuierlich verschoben und es werden nur zu gewissen Zeitpunkten während der Fokusverschiebung Laserstrahlungspulse abgegeben. Die entsprechenden Optiken bzw. Einrichtungen zu Fokusverschiebungen arbeiten dennoch kontinuierlich, weshalb unter dem Begriff "Fokusverschiebung" hier auch die entsprechende Verschiebung des Punktes verstanden wird, an den optische Strahlung fokussiert würde, auch wenn eine solche Strahlung momentan nicht abgegeben wird, z. B. zwischen zwei Laserpulsen.

Die hohe Fokussierung der Laserstrahlung, d. h. ein geometrisch stark begrenzter Fokus ist für nicht lineare Wirkungen von großer Bedeutung, da nur dann die erforderlichen Leistungsdichten im Material erreicht werden können. Dies gilt sowohl für nicht lineare Prozesse, bei denen ein einzelner Fokus bereits eine Wechselwirkung zu Folge hat, als auch für Prozesse, bei denen mehrere Laserstrahlungspulse, die hintereinander abgegeben werden, zusammenwirken, um einen Material trennenden Effekt zu erreichen. Es sind diesbezüglich auch Ansätze bekannt, bei denen an mehreren überlappenden Fokusstellen Laserstrahlungspulse abgegeben werden und erst im Überlappungsbereich die Zusammenwirkung der mehreren Laserstrahlungspulse zur Materialtrennung führt.

Dreidimensionale Schnittflächen, die sich parallel zur optischen Achse des Strahleinfalls (der sog. Hauteinfallsrichtung) erstrecken, sind z. B. als zylindermantelförmige Schnittflächen im Gebiet der Ophthalmologie, insbesondere bei der Katarakt-Operation, gefordert. Hier wird eine kreisrunde Öffnung in der Vorderseite des Kapselsacks mit einem bestimmten Durchmesser erzeugt. Die Schnittflächenform ist dann ein Kreiszylinder, welcher ungefähr parallel zur optischen Achse und somit auch parallel zur Haupteinfallsrichtung der optischen Strahlung orientiert ist.

Die EP 1486185 betrifft ein Gerät für die Katarakt-Operation, bei dem die Laserstrahlung mit Hilfe einer optischen Lichtleitfaser zum Handteil geleitet wird. Im Handteil wird ein Kollimator mechanisch verstellt, um die Lage des Fokus längs der optischen Achse zu verstellen. Die Fokusverstellung längs der optischen Achse kann deshalb nur sehr langsam erfolgen, da eine schnelle Bewegung des Kollimators unerwünschte Vibrationen und eine thermische Belastung des Handteiles zur Folge hätte. Darüber hinaus ist die Verwendung einer Lichtleitfaser bei den Leistungen, die zur Augenchirurgie nötig sind, äußerst problematisch.

In den US 7486409 und US 6590670 wird eine Methode zur schnellen Variation der Tiefenlage eines Fokus auf Basis einer schwingenden Stimmgabel beschrieben. Dabei wird mindestens eine Linse einer optischen Anordnung an den vibrierenden Arm einer Stimmgabel befestigt, welche durch einen Elektromagnet in Schwingungen versetzt wird. Die DE 10034251 schlägt zur Tiefenverstellung vor, einen Eckreflektor am schwingenden Arm einer Stimmgabel anzubringen. Der Eckreflektor wird mit einem nicht-kollimierten Lichtbündel beleuchtet, und durch Verstellung der Lage des Eckreflektors wird die Ausbreitung des rückreflektierten Lichtbündels variiert. Fokussiert man dieses Lichtbündel mit einem Objektiv, erhält man eine schnelle Variation der Fokuslage in Tiefenrichtung, d.h. längs der optischen Achse. Diese Stimmgabelanordnungen wurden für die optische Messtechnik vorgeschlagen.

Zum Erzeugen dreidimensionaler Schnittflächen, die sich parallel zur optischen Achse des Strahleinfalls erstrecken, sind Laserbearbeitungsvorrichtungen bekannt, welche eine Optik sowie eine Einrichtung zur dreidimensionalen Fokusverstellung aufweisen. Solche Bearbeitungsvorrichtungen verstellen den Fokus im Bildfeld und ergänzen diese zweidimensionale Verstellung durch eine Verstellung der Bildfeldebene zur dreidimensionalen Fokuslageneinstellung. Da die Verstellung der Bildfeldebene sehr viel langsamer ist, als die zweidimensionale Fokuslagenverstellung in der Bildebene, wird mit solchen Geräten darauf geachtet, dass die Bildebenenverstellung möglichst wenig benötigt wird, um die Schnittflächenerzeugung möglichst schnell zu gestalten. Es sind deshalb aus dem Stand der Technik beispielsweise spiralförmige Bahnkurven bekannt, welche eine schnelle Verstellung in der Bildebene mit einer vergleichsweise langsamen Verschiebung oder Verstellung der Bildebenenlage kombinieren. Auf diese Weise können beispielsweise zylinderförmige Schnittflächen, die parallel zur optischen Achse liegen, sehr schnell erzeugt werden.

Nachteilig an diesem Ansatz ist es, dass die Optik so gestaltet werden muss, dass innerhalb des Bildfeldes eine schnelle zweidimensionale Fokuslagenverstellung möglich ist. Auch muss die Bildfeldgröße so gestaltet werden, dass gewünschte Schnittflächengrößen abgedeckt werden.

Der Erfindung liegt deshalb die Aufgabe zu Grunde, die Verfahren bzw. Bearbeitungsvorrichtung der eingangs genannten Art so weiterzubilden, dass aufwandsgering Schnittflächen erzeugt werden können, die im Wesentlichen parallel zur optischen Achse verlaufen, insbesondere zylindermantelförmige Schnittflächen.

Die Erfindung ist in den unabhängigen Ansprüchen definiert. Die abhängigen Ansprüche betreffen Weiterbildungen.

Die Erfindung setzt eine Bahnkurve zur Schnittflächenerzeugung ein, welche vom üblichen Ansatz, der eine axiale Verstellung des Fokus möglichst zu minimieren sucht, grundlegend abweicht. Vielmehr wird nun die Lage des Fokus quer zur optischen Achse (sog. laterale Verstellung) sehr viel langsamer verstellt als axial, indem die Fokuslage auf der Kurve geführt wird, welche die Schnittfläche in Projektion längs der optischen Achse hat. Diese Kurve ist in Ausführungsformen die Aufrisslinie der Schnittfläche. Es handelt sich bevorzugt um eine geschlossene Kurve, z. B. eine periodische Lissajous-Figur, beispielsweise einen Kreis, eine Ellipse etc. Senkrecht dazu, d. h. axial bzw. längs der optischen Achse wird der Fokus oszillierend verstellt. Man erhält somit eine Bahnkurve, welche in einer senkrecht zur optischen Achse gesehenen Draufsicht auf die Schnittfläche sich mäanderförmig zwischen dem unteren und dem oberen Rand der Schnittfläche entsprechend der unteren und der oberen axialen Fokusposition hin und her bewegt.

Die Schnittfläche, für welche die Steuerdaten im Verfahren erzeugt werden und welche das Steuergerät der Bearbeitungsvorrichtung einstellt, erstreckt sich, wie bereits erwähnt, nicht zwingend strikt, sondern ggf. nur im Wesentlichen parallel zur optischen Achse. Abweichungen von der Parallelität die klein gegen die Länge der Kurve sind, sind zulässig. Insbesondere bei Lasereinrichtungen, die ein gegenüber der Ausdehnung des zu bearbeitenden Gebietes kleines Bildfeld haben und mit einer Bildfeldverstellung arbeiten, ist es möglich, innerhalb dieses kleinen Bildfeldes eine zusätzlich Fokusverstellung auszuführen. Diese erlaubt es natürlich, während das Bildfeld entlang der Kurve bewegt wird, die Lage des Fokus zusätzlich lateral auszulenken. Die Schnittfläche kann dadurch Bereiche haben, in denen keine strikte Parallelität zur optischen Achse gegeben ist. Gleichermaßen ist es möglich, eine zumindest abschnittsweise gegenüber der optischen Achse geneigte Schnittfläche zu erzeugen, indem die zusätzliche Fokusverstellung innerhalb des Bildfeldes so angesteuert wird, dass insgesamt die Schnittfläche leicht schräg liegt. In diesem Fall wird die zusätzliche laterale Fokusverstellung innerhalb des Bildfeldes geeignet zur oszillierenden axialen Fokusverstellung synchronisiert.

Die Bewegung des Fokus erfordert nicht zwingend, dass auf jede Fokusposition optische Strahlung zur Materialtrennung abgegeben wird. So ist es beispielsweise bei der Verwendung gepulster Strahlung sehr viel einfacher, den Fokus kontinuierlich zu verschieben, so dass die Spots auf die Laserstrahlungspulse abgegeben werden, im Material entsprechend der Bahngeschwindigkeit des Fokus beabstandet sind. Setzt man als materialtrennenden Effekt einen optischen Durchbruch ein, welcher eine Plasmablase erzeugt, ist es sogar bevorzugt, einen gewissen Abstand zwischen den Spots, auf die jeweils ein Laserstrahlungspuls abgegeben wird, einzuhalten. Es sind aber auch Verfahren bekannt, bei denen mehrere Laserstrahlungspulse zur Materialtrennung zusammenwirken, ohne einen optischen Durchbruch zu erzeugen. Diese Ansätze werden auch als Sub-Threshold-Verfahren bezeichnet.

Weiter schließt die Verstellung des Fokus gemäß der geschilderten Bahnkurve nicht aus, dass in einzelnen Abschnitten der Bahn, auf welcher der Fokus geführt wird, keine Laserstrahlung abgegeben wird, welche einen Material trennenden Effekt hat. Hier sind insbesondere bei der Verwendung einer plasmablasenbasierten Materialtrennung Abschnitte der Bahnkurve relevant, auf denen der Fokus von oben nach unten, d. h. in der Einfallsrichtung der optischen Strahlung bewegt wird. Erzeugt man Plasmablasen zur Materialtrennung, können in Gebieten, unterhalb der sich bildenden Plasmablase (die durchaus größer sein kann als der Fokus) zumindest für eine Zeitdauer keine weiteren optischen Durchbrüche und Plasmablasen erzeugt werden, da jede darüber liegende Plasmablase die Fokusqualität derart schwächt, dass ein optischer Durchbruch nicht mehr gesichert erreicht werden kann. Es ist deshalb im Rahmen der Erfindung durchaus möglich, in denjenigen Abschnitten der Kurve, in denen der Fokus nach unten, d. h. von der Lasereinrichtung wegbewegt wird, die optische Strahlung dunkelzutasten, d. h. abzuschalten oder zumindest hinsichtlich ihres Material bearbeitenden Effektes zu deaktivieren. Für solche Deaktivierungen sind im Stand der Technik verschiedene Ansätze bekannt, beispielsweise eine Laserpulsverlängerung, eine Fokusverschlechterung, spektrale Änderungen, Änderungen der Polarisation etc. Es ist deshalb im Rahmen der Erfindung ein Verfahren zur Erzeugung von Steuerdaten vorteilhaft, das auf

Abschnitten der Bahnkurve, in denen sich die Lage des Fokus mit (d. h. in gleicher Richtung) einer Einfallsrichtung der optischen Strahlung bewegt, die optische Strahlung abgeschaltet oder so modifiziert wird, dass die optische Strahlung keine Materialtrennungswirkung im transparenten Material hat.

Um die Schnittfläche auch in solchen Fällen möglichst dicht mit Abschnitten der Bahnkurve, in denen die optische Strahlung Materialtrennungswirkung hat, zu belegen, ist es zu bevorzugen, die Oszillation asymmetrisch zu gestalten, so dass Abschnitte der Bahnkurve, in denen sich die Lage des Fokus mit der Einfallsrichtung der optischen Strahlung bewegt, steiler verlaufen, als Abschnitte der Bahnkurve, in denen sich die Lage des Fokus entgegen der Einfallsrichtung der optischen Strahlung bewegt.

Die Schnittfläche kann im mathematischen Sinne zweifach zusammenhängend sein. Die Kurve ist dann geschlossen. Die erzeugte Schnittfläche kann insbesondere zylindermantelförmig sein, was bei Anwendungen an der erwähnten Katarakt-Operation vorteilhaft ist. Gleichermaßen kann die Schnittfläche natürlich auch zur Sektionierung oder Veränderung von transparentem Material, beispielsweise Augengewebe eingesetzt werden. In Frage kommen unter anderem die Sektionierung der Augenlinse vor der Entfernung bei der Katarakt-Operation oder die gezielte Schwächung der Kornea mit dem Ziel, dadurch die Hornhautkrümmung fehlsichtigkeitskorrigierend zu modifizieren. Insbesondere aber nicht ausschließlich für diese Anwendungen sind geschlossene, d. h. periodische Lissajous-Figuren geeignet, die sich kreuzen. Solche Lissajous-Figuren können erreicht werden, indem eine zweiachsige Ablenkung gemäß harmonischen Funktionen ausgeführt wird, die auf ganzzahligen Vielfachen einer Grundfunktion beruhen, wobei die ganzzahligen Vielfachen für die zwei Ablenkachsen unterschiedlich sind.

Die Schnittfläche kann aber auch einfach zusammenhängend sein; die Kurve ist dann eine nicht geschlossene Linie.

Wird an den axial oberen bzw. unteren Fokuspositionen eine Materialtrennung ausgeführt, definieren diese Fokuspositionen automatisch den oberen bzw. unteren Rand der Schnittfläche.

Die Verschiebung des Bildfeldes erlaubt einen Verzicht auf aufwendige Optiken, deren Bildfeld so groß ist, dass es das gesamte Bearbeitungsgebiet abdeckt. Es sei allerdings darauf hingewiesen, dass es durchaus möglich ist, die Bearbeitungsvorrichtung bzw. die Verfahren so zu modifizieren, dass die Verstellung der Lage des Fokus quer zur optischen Achse ohne Bildfeldverschiebung auskommt, wenn die Bildfeldgröße größer ist als die Maximalgröße der beabsichtigten, angestrebten oder erzeugten Schnittfläche. Derartiges liegt im Rahmen der Erfindung. Man hat dann immer noch den Vorteil, dass die Fokusverstellung quer zur optischen Achse sehr viel langsamer ausgeführt werden kann, als längs der optischen Achse. Für die Anwendung in der Ophthalmologie sollte das Bildfeld im Auge dann einen Durchmesser von ca. 5 mm mindestens erreichen.

Verwendet man kleinere Bildfelder und möchte dennoch Strukturen erzeugen, die eine Maximalausdehnung quer zur optischen Achse haben, welche größer ist als die Bildfeldgröße, kann die Verschiebung des Bildfeldes besonders einfach durch eine Bewegung der Optik oder einen optischen Elementes quer zur optischen Achse bewirkt werden. Dies kann in Kombination mit einer lateralen Fokusverstellung innerhalb des Bildfeldes erfolgen.

Die Vorrichtung sowie das Verfahren zur Erzeugung einer Schnittfläche sind besonders bei der Katarakt-Operation vorteilhaft anwendbar. Für solche Operationen benötigt man ein bewegliches Handteil, das am Auge aufgesetzt wird. Es ist deshalb bevorzugt, dass die Lasereinrichtung ein Basisteil und ein bewegliches Handteil aufweist, die miteinander über eine flexible oder gelenkige Übertragungseinrichtung verbunden sind, wobei die Einrichtung zur Verstellung der Lage des Fokus zweiteilig ausgebildet ist und einen ersten Scanner, der die Lage des Fokus längs der optischen Achse verstellt und im Basisteil angeordnet ist, und einen zweiten Scanner aufweist, der die Lage des Fokus quer zur optischen Achse verstellt und von dem zumindest eine Komponente im Handteil angeordnet ist. Für den zweiten Scanner zur Verstellung der Lage des Fokus quer zur optischen Achse ist diese Komponente bevorzugt ein Stellglied zur Verschiebung eines Objektivs quer zur optischen Achse.

Die Übertragungseinrichtung kann bevorzugt als Freistrahloptik ausgebildet sein. Es hat sich als vorteilhaft herausgestellt, wenn das Basisteil in die Übertragungseinrichtung ein nicht-kollimiertes Strahlenbündel einkoppelt, also ein Strahlenbündel, das einen bestimmten Divergenz- bzw. Konvergenzzustand hat. Die Übertragungseinrichtung, beispielsweise ein entsprechender Gelenkarm, führt dieses Strahlenbündel unter Beibehaltung des Divergenz- bzw. Konvergenzzustandes vom Basisteil zum Handteil. Die Querverschiebung des Objektives zur Realisierung des xy-Scanvorgangs ist dann kombiniert mit einer entsprechenden Aktivierung einer Divergenzverstelleinrichtung, welche die Divergenz oder Konvergenz des nicht-kollimierten Bündels beeinflusst, um eine Weglängenänderung, welche sich durch die Querverschiebung des Objektivs ergibt, zu kompensieren. Damit ist verhindert, dass die Querverschiebung des Objektivs, d.h. die Bildfeldverstellung zum xy-Scannen nicht unerwünscht eine Verstellung der Fokuslage längs der optischen Achse mit sich bringt.

Die Divergenzverstelleinrichtung kann besonders bevorzugt im Basisteil angeordnet werden, da dann das Handteil kompakt bleibt. Sie kann als Teleskop mit einem diesen verstellenden Aktuator realisiert sein.

Die Verwendung eines nicht-kollimierten Strahlengangs zwischen Basisteil und Handteil hat weiter den Vorteil, dass besonders einfach der z-Scanner realisiert werden kann, indem der erste Scanner die Weglänge des nicht-kollimierten Strahlengangs verstellt. Für diese Weglängenverstellung kann ein Retro-Reflektor, beispielsweise ein Eckspiegel verwendet werden. Erst der Einsatz des nicht-kollimierten Strahlengangs erlaubt eine einfache Verstellung der Tiefenlage des Fokus durch Weglängenveränderung des Strahlengangs zum Objektiv. Dafür ist es allerdings wesentlich, dass zwischen dem ersten Scanner und dem Objektiv kein Parallelstrahlengang besteht, da dieser die Wirkung eines solchen ersten Scanners zunichtemachen würde. Der Strahlengang vom z-Scanner bis zum Objektiv ist folglich nichttelezentrisch.

Bevorzugt umfasst das Basisteil einen nicht-kollimierten, d. h. divergierenden oder konvergierenden Strahlengangabschnitt und der erste Scanner umfasst einen Eckspiegel, der in diesem Strahlengangabschnitt liegt. Zur Verstellung der Lage des Fokus längs der optischen Achse wird der Eckspiegel verschoben, um die Länge des nicht-kollimierten Strahlengangabschnitts zu verändern.

Die Verschiebung des Eckspiegels kann besonders schnell durch einen Schwinger erfolgen, der den Eckspiegel in Vibration zur periodischen Veränderung der Länge des nicht-kollimierten Strahlengangabschnittes versetzt. Alternativ ist es möglich, mehrere Eckspiegel auf einer rotierenden Scheibe zu montieren, die nacheinander im nicht-kollimierten Strahlengangabschnitt durchlaufen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen. Auch betrifft die Beschreibung von Verfahrensmerkmalen zur Erzeugung von Steuerdaten gleichermaßen eine entsprechende Ausgestaltung der Steuereinrichtung, welche die Bearbeitungseinrichtung steuert. Umgekehrt sind Merkmale, welche hinsichtlich der Bearbeitungsvorrichtung insbesondere deren Steuereinrichtung beschrieben sind, gleichermaßen relevant für das entsprechende Verfahren zur Steuerdatenerzeugung.

Die Steuerdatenerzeugung kann separat, d. h. unabhängig von der Bearbeitungsvorrichtung ausgeführt werden. Sie setzt natürlich entsprechende Kenntnis über die Bearbeitungsvorrichtung voraus, für welche die Steuerdaten vorgesehen werden.

Nachfolgend wird die Erfindung beispielsweise anhand der beigefügten Zeichnungen, die auch erfindungswesentliche Merkmale offenbaren, noch näher erläutert. Es zeigen:

Fig. 1    eine Schemadarstellung einer Behandlungsvorrichtung für ophthalmologische Eingriffe, insbesondere zur Fehlsichtigkeitskorrektur,

Fig. 2    eine Schemadarstellung hinsichtlich des Aufbaus des Behandlungsgerätes der Fig. 1,

Fig. 3    eine Prinzipdarstellung zur Einbringung gepulster Laserstrahlung in das Auge mit dem Behandlungsgerät der Fig. 1,

Fig. 4    eine schematische Darstellung für eine Schnittfläche durch einen Kapselsack eines Auges,

Fig. 5    eine Schemadarstellung der in Fig. 4 dargestellten Schnittfläche,

Fig. 6    eine Schemadarstellung ähnlich der Fig. 5 zur Veranschaulichung der Führung des Laserstrahls auf einer Bahnkurve,

Fig. 7    eine Schemadarstellung einer Schnittfläche, die anders als die Schnittfläche der Fig. 4 kein Gebiet umschließt,

Fig. 8    eine Schemadarstellung ähnlich der Fig. 4 zur Verdeutlichung einer möglichen Anwendung der Schnittfläche der Fig. 7,

Fig. 9    eine Schemadarstellung eines Behandlungsgeräts, das zur Katarakt-Operation ausgebildet ist,

Fig. 10    eine Schemadarstellung eines Handteils des Behandlungsgeräts der Fig. 9,

Fig. 11    eine Schemadarstellung eines z-Scanners des Behandlungsgerätes der Fig. 9,

Fig. 12    eine schematische Blockdarstellung für das Steuerkonzept des Behandlungsgerätes der Fig. 9, und

Fig. 13    und 14 Schemadarstellungen für alternative Ausgestaltungen des z-Scanners des Behandlungsgeräts der Fig. 9.

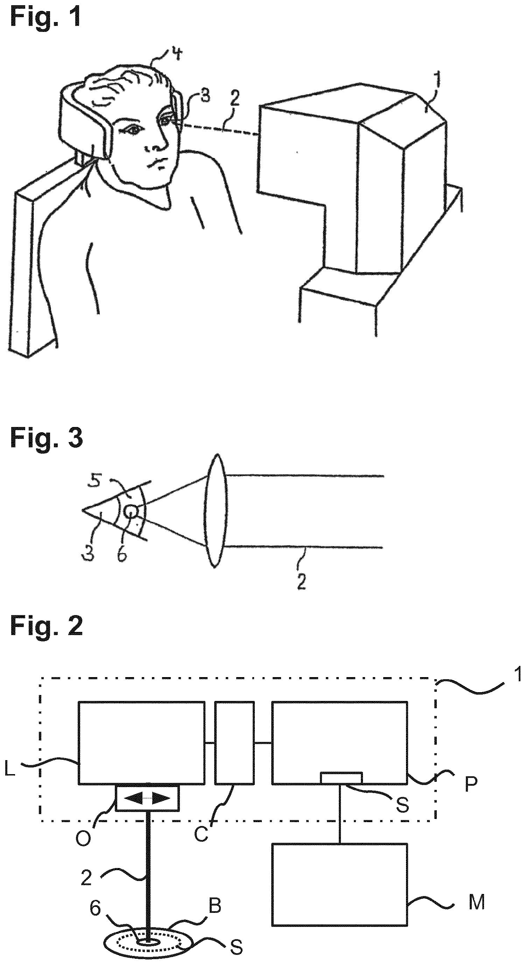

Fig. 1 zeigt ein Behandlungsgerät 1 für die Augenchirurgie. Damit kann z. B ein augenchirurgische Verfahren ausgeführt werden, das dem in EP 1 159 986 A2 bzw. US 5,549,632 beschriebenen ähnelt. Das Behandlungsgerät 1 erzeugt mittels Behandlungs-Laserstrahlung 2 eine Materialtrennung in transparentem Material. Diese Materialtrennung kann z. B. eine Schnittflächenerzeugung sein, insbesondere kann das Behandlungsgerät zur Fehlsichtigkeitskorrektur eine Veränderung an einem Auge 3 eines Patienten 4 bewirken. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Aberrationen höherer Ordnung umfassen. Die Materialtrennung kann auf dem Gebiet der Hornhautchirurgie aber auch an anderen Geweben des Auges eingesetzt sein, z. B. bei der Kataraktoperation. Soweit nachfolgend Bezug auf die Augenchirurgie genommen wird, ist das auf jeden Fall nur exemplarisch und nicht einschränken zu verstehen.

Die Baugruppen des Gerätes 1 werden im beschriebenen Ausführungsbeispiel von einer integrierten Steuereinheit gesteuert, die aber natürlich auch eigenständig ausgebildet sein kann.

Fig. 2 zeigt schematisch das Behandlungsgerät 1. Es weist in dieser Variante mindestens drei Einrichtungen oder Module auf. Eine Lasereinrichtung L gibt über eine Optik O den Laserstrahl 2 auf das Material, z. B. ein Auge ab und verstellt die Lage des Fokus im Material in drei Raumrichtungen. Die Verstellung längs der Haupteinfallsrichtung der optischen Strahlung (z-Achse) wird axiale Verstellung bezeichnet, die Verstellung senkrecht dazu als laterale Verstellung. In der in Fig. 2 gezeigten Variante hat die Optik O ein Bildfeld B, in dem ein Fokus 6 der Laserstrahlung 2 liegt, das kleiner ist als die Ausdehnung des zu bearbeitenden Gebietes. Zur lateralen Verstellung der Lage des Fokus 6 im Material wird das Objektiv O der Lasereinrichtung L quer zur optischen Achse verschoben wird. Dies ist in Fig. 2 durch einen Doppelpfeil für das Objektiv O angedeutet. Dadurch wird der Fokus 6 samt dem Bildfeld B verschoben. Optional kann der Fokus 6 zusätzlich in einem Bereich S innerhalb des Bildfeldes B feinverstellt werden.

In einer alternativen Variante hat die Lasereinrichtung L eine laterale Scaneinrichtung, welche den Fokus 6 im Bildfeld B verschiebt, das so groß ist, das es die Ausdehnung des zu bearbeitenden Gebietes überdeckt. Zusätzlich ist eine axiale Scaneinrichtung vorgesehen.

Der Betrieb der Lasereinrichtung L erfolgt für alle Varianten vollautomatisch unter Steuerung durch eine integrierte oder separate Steuereinrichtung C. Die Lasereinrichtung L startet auf ein entsprechendes Startsignal hin die Ablenkung des Laserstrahls 2 und erzeugt dadurch Schnittflächen auf noch zu beschreibende Art und Weise.

Die Steuereinrichtung C arbeitet nach Steuerdaten, die entweder von ihr erzeugt wurden oder ihr zugeführt wurden. In letzterem Fall, der in Fig. 2 gezeigt ist, werden die für den Betrieb erforderlichen Steuerdaten der Steuereinrichtung C zuvor von einer Planungseinrichtung P als Steuerdatensatz über nicht näher bezeichnete Steuerleitungen zugeführt. Die Ermittlung oder Übertragung der Steuerdaten findet vor dem Betrieb der Lasereinrichtung L statt. Natürlich kann die Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

In der Augenheilkunde wird bevorzugt vor dem Einsatz des Behandlungsgerätes 1 die Fehlsichtigkeit des Auges 3 mit einer oder mehreren Messeinrichtungen M vermessen. Die Messwerte werden dann dem Steuereinrichtung bzw. der Planungseinrichtung P zugeführt und bilden die Basis für die Steuerdatenerzeugung. Insbesondere kann die Lage und/oder Ausdehnung eines zu bearbeitenden, insbesondere zu sektionierenden Bereichs gemessen werden.

Die Steuereinrichtung bzw. die Planungseinheit P erzeugt den Steuerdatensatz aus den Messdaten, die ermittelt wurden, z. B. für das zu behandelnde Auge. Sie werden der Planungseinheit P über eine Schnittstelle S zugeführt und stammen im dargestellten Ausführungsbeispiel aus einer Messeinrichtung M, die das Auge des Patienten 4 zuvor vermessen hat. Natürlich kann die Messeinrichtung M auf beliebige Art und Weise die entsprechenden Messdaten an die Planungseinrichtung P oder direkt an die Steuereinrichtung C übermitteln.

Die Laserstrahlung 2 wird in der beschriebenen Ausführungsform als gepulster Laserstrahl fokussiert in das Material, z. B. das Auge 3 gebracht. Die von der Lasereinrichtung L erzeugte Pulsdauer liegt dabei z. B. im Femtosekundenbereich, und die Laserstrahlung 2 wirkt mittels nicht-linearer optischer Effekte im Material, z. B. dem Kapselsack der, der Augenlinse oder der Hornhaut. Der Laserstrahl weist z. B. 50 bis 800 fs kurze Laserpulse (bevorzugt 100 - 400 fs) mit einer Pulswiederholfrequenz zwischen 10 kHz und 10 MHz auf. Die Art des Material trennenden Effektes, den das Behandlungsgerät 1 mit der Laserstrahlung 2 nutzt, ist jedoch für die nachfolgende Beschreibung nicht weiter relevant, insbesondere muss keine gepulste Laserstrahlung verwendet werden. Wesentlich ist lediglich, dass ein Fokus von Bearbeitungsstrahlung 2 im Material längs einer Bahnkurve verschoben wird. Alternativ kann UV-Strahlung (300 bis 400 nm), insbesondere mit einer Wellenlänge von ca. 355 nm und einer Pulsdauer zwischen 0,1 und 10 ns verwendet werden.

Das Behandlungsgerät 1 bildet im Material eine Schnittfläche aus, deren Form vom Muster abhängt, mit dem die Laserpulsfoki im Gewebe angeordnet sind/werden. Das Muster hängt wiederum von der Bahnkurve ab, entlang der der Fokus verschoben wird. Sie gibt Zielpunkte für die Fokuslage vor, an denen ein oder mehrere Laserpuls(e) abgegeben wird(werden), und definiert letztlich die Form und Lage der Schnittfläche.

Eine mögliche Wirkungsweise des Laserstrahls 2 ist in Fig. 3 schematisch angedeutet. Er wird mittels einer nicht näher bezeichneten Optik der Lasereinrichtung L in das Material, z. B. die Hornhaut 5 oder Linse des Auges fokussiert. Dadurch entsteht im Material ein Fokus 6, in dem die Laserstrahlungsenergiedichte so hoch ist, dass in Kombination mit der Pulslänge ein nicht-linearer Effekt auftritt. Beispielsweise kann jeder Puls der gepulsten Laserstrahlung 2 am jeweiligen Ort des Fokus 6 einen optischen Durchbruch im Material, z. B. in der Hornhaut 5 oder Linse erzeugen, welcher in Fig. 3 exemplarisch durch eine Plasmablase schematisch angedeutet ist. Dadurch wird aufgrund dieses Laserpulses Material, z. B. Gewebe getrennt. Bei Entstehung einer Plasmablase umfasst die Gewebeschichttrennung ein größeres Gebiet, als den Spot, welchen der Fokus 6 der Laserstrahlung 2 überdeckt, obwohl die Bedingungen zur Erzeugung des Durchbruches nur im Fokus erreicht werden. Damit von jedem Laserpuls ein optischer Durchbruch erzeugt wird, muss die Energiedichte, d. h. die Fluence der Laserstrahlung oberhalb eines gewissen wellenlängsabhängigen und pulslängenabhängigen Schwellwertes liegen. Dieser Zusammenhang ist dem Fachmann beispielsweise aus der DE 695 00 997 T2 bekannt.

Alternativ kann ein materialtrennender Effekt durch die gepulste Laserstrahlung auch dadurch erzeugt werden, dass mehrere Laserstrahlungspulse im einen Bereich abgegeben werden, wobei sich für mehrere Laserstrahlungspulse die Spots 6, d.h. Orte des Fokus 6 überlappen. Es wirken dann mehrere Laserstrahlungspulse zusammen, um einen gewebetrennenden Effekt zu erreichen, ohne dass Plasmablasen entstehen (sog. Sub-threshold-Verfahren). Beispielsweise kann das Behandlungsgerät 1 das Prinzip verwenden, das in der WO 2004/032810 A2 beschrieben ist.

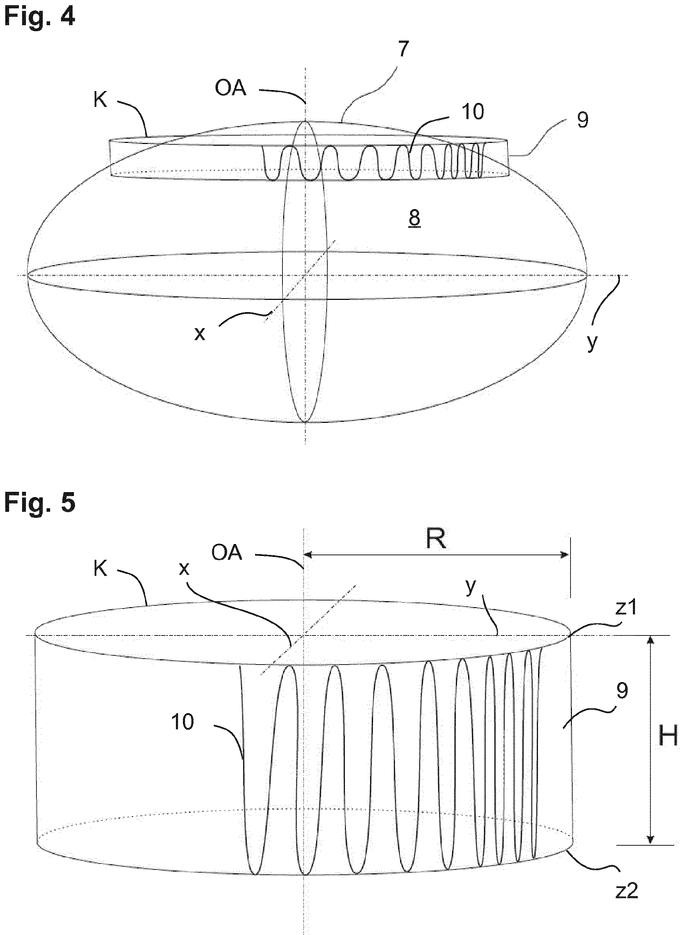

Fig. 4 zeigt exemplarisch die Erzeugung einer Schnittfläche 9 in einem Kapselsack, der eine Linse 8 des Auges 3 einhüllt. Die Schnittfläche 9 ist zweifach zusammenhängend. Sie fällt insofern nicht unter die Ansprüche, als sie die Form eines Kreiszylindermantels hat, also der Mantelfläche eines Zylinders, dessen Erzeugende eine kreisförmige Kurve K ist; jedoch sind auch andere geschlossene Figuren als Erzeugende möglich, insbesondere jede periodische Lissajous-Figur, auch sich kreuzende.

Die Kurve K liefert die Vorgabe für die laterale Verstellung des Fokus, also in der x/y-Ebene. Die entsprechenden Koordinaten x/y liegen in der Ebene des Bildfeldes B der Lasereinrichtung L. Sie sind in den Figuren exemplarisch eingetragen. Senkrecht zur optischen Achse OA, welche die Haupteinfallsrichtung der optischen Laserstrahlung 2 ist, - also axial - wird die Lage des Fokus 6 entlang der Kurve K verstellt. Währenddessen wird die axiale Lage des Fokus zugleich längs der optischen Achse OA, d. h. senkrecht zur x/y-Ebene, oszillierend verstellt. Dadurch ergibt sich eine Bahnkurve 10 in der Schnittfläche 9, die zwischen einer oberen axialen Fokusposition z1 und einer unteren axialen Fokusposition z2 hin und her oszilliert. Diese Oszillationen werden während der Bewegung entlang der Kurve K mehrfach ausgeführt.

Dieses Vorgehen vermeidet eine schnelle laterale Ablenkung der Laserstrahlung 2 über das zu bearbeitende Gebiet, welches im beschriebenen Fall durch den Radius R der Kurve K charakterisiert ist und eine laterale Maximalabmessung von 2*K hat. Optional wird eine Größe des Bildfelds B verwendet, die sehr viel kleiner ist als diese laterale Ausdehnung. Grundsätzlich wird eine schnelle axiale Bewegung ausgeführt, während die Verschiebung in der x/y-Ebene der Kurve K folgt. Somit werden im wesentlichen Schnitte erzeugt, die parallel zur optischen Achse liegen. Damit kann ein einfaches optisches System verwendet werden, das keiner schnellen lateralen Fokusverschiebung mit großer Amplitude bedarf.

Für die axiale Fokusverschiebung kommen mehrere Ansätze in Frage, beispielsweise eine elektrooptische Linse oder ein sogenannter zweistufiger z-Scanner, der eine langsame großhubige Verstellung mit einer schnellen kurzhubigen Verstellung kombiniert. Die beiden Stufen eines solchen zweistufigen z-Scanners können räumlich getrennt oder kombiniert ausgebildet sein.

Für den Fall der Bearbeitung der menschlichen Linse, die nachfolgend exemplarisch abgehandelt werden soll, ist ein maximaler Wert NA = 0,2 aus anatomischen Gründen nicht wesentlich überschreitbar. Daraus ergibt sich die effektive Brennweite des verwendeten Objektivs zu mindestens etwa

$$f \approx \frac{n \cdot D}{2 \cdot NA} \approx \frac{1.3 \cdot 6mm}{2 \cdot 0.2} \approx 19mm$$

Um die Bahnkurve 10 abzufahren, muss der die axiale Verstellung eine Bahn beschreiben gemäß

$$\zeta(t) = \zeta_{max} \sin(\varpi \cdot t).$$

Für die Beschleunigung erhält man entsprechend

$$\ddot{\zeta}(t) = -\varpi^2 \cdot \zeta_{max} \sin(\varpi \cdot t)$$

Der für die Kurve K typische Radius R beträgt bei der Katarakt-Operation zwischen 2 und 3 mm. Damit beträgt der Umfang der Schnittfläche etwa 20 mm. Um eine gute Materialtrennung bei einer plasmablasenbasierten Technik zu erzielen, sollte der tangentiale Bahnabstand (benachbarte axiale Oszillationen) zwischen 1 und 10 $\mu$m betragen. Es sind also etwa zwischen 2.000 und 20.000 vertikale Schnittbahnen zu erzeugen. Innerhalb der Schnittbahnen sollten die Spotabstände in der Größenordnung zwischen 1 und 10 $\mu$m liegen.

Die Höhe H muss mindestens der Kapselsackdicke, also ca. 20 bis 25 $\mu$m entsprechen. Wenn sie geringer ist, können mehrere Schnittflächen 9 aufeinander "gestapelt" werden, um eine komplette Durchtrennung des Kapselsackes zu erreichen, wobei ein gewisser Überlapp sinnvoll sein kann.

Praktikabel erscheint eine Gesamthöhe von 25 $\mu$m bis 250 $\mu$m. Damit hat die Schnittfläche 9 einen Flächeninhalt von ca. 500.000 bis 5.000.000 gm$^2$. Mit einer Gitterkonstanten von $1 \times 1$ μm bis maximal 10x10 μm werden also 50.000 bis 5.000.000 Laserspots platziert.

Bei einer Laserpulswiederholrate von 100 kHz ergibt sich eine minimale Schnittflächenerzeugungszeit (ohne Pausen, Totzeiten) von 0,5 bis 50 s. Da bei einer solchen Laserpulswiederholrate Pulsenergien im μJ-Bereich leicht erzeugbar sind, ist es bevorzugt, einen größeren mittleren Spotabstand mit höherer Energie zu kombinieren, z. B. 0.5 μJ und 3x3 μm. Damit beträgt die Schnittflächenerzeugungszeit auch bei großer Zylinderhöhe nur wenige Sekunden (weniger als 10 s).

Alternativ kann mit geringer Pulsenergie (<100 nJ) und einer Laserpulswiederholrate im Bereich einiger MHz ein Spotabstand von $1 \times 1$ μm verwendet werden. Beispielsweise ergibt sich bei 5 MHz ebenfalls wiederum eine Schnittflächenerzeugungszeit von nur wenigen Sekunden (weniger als 10 s).

Dies bedeutet, dass die axiale Verstellung innerhalb von ca. 5 Sekunden beim lateralen Umlauf um die Kurve K mit einem Umfang von 20 mm je nach Bahnabstand 2.000 bis 20.000 axiale Oszillationen realisieren muss. Die axiale Frequenz (Schwingungsfrequenz) beträgt damit 500 Hz bis 5 kHz. Für die Beschleunigung ergibt sich

$$Max(\ddot{\zeta}(t)) = \varpi^2 \cdot \zeta_{max} = 4\pi^2 f^2 \cdot \zeta_{max} \approx 10^{6...11}\, s^{-2} \cdot \zeta_{max}$$

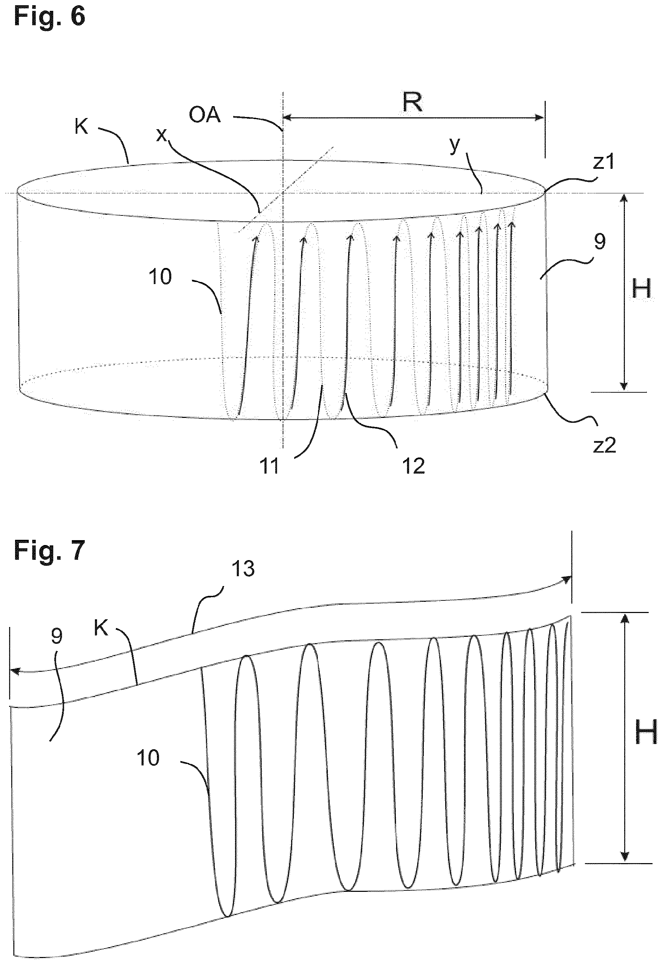

Zu beachten ist, dass die Art der Bahnkurve 10 eine ungünstige Beeinflussung des optischen Strahlweges durch das Material (Gewebe) durch vorangegangene Wechselwirkungsvorgänge nicht automatisch verhindert, wie dies bei einem schichtweisen Aufbau der Schnittfläche längs der Einfallsrichtung der Laserstrahlung der Fall wäre. Deshalb ist gemäß Fig. 6 eine Weiterbildung vorgesehen, indem die Laserstrahlung 2 auf Abschnitten 11 der Bahnkurve 10 in die Tiefe des Materials (weg von der Einfallsrichtung) so modifiziert wird, dass keine die Transmission nachfolgender Pulse nachteilig beeinflussende Wechselwirkung erfolgt, oder diese zumindest verringert wird. Im einfachsten Fall werden die Pulse auf diesen Abschnitten 11 vollständig unterdrückt. Dieses Konzept kann auch noch für die Bereiche in der Nähe der Umkehrpunkte der Bahn ausgedehnt werden, also bis zu etwa 40 % in der Umgebung der Wendepunkte der Bahnkurve 10 (nicht notwendigerweise symmetrisch).

Fig. 7 zeigt exemplarisch eine Schnittfläche 9, die im mathematischen Sinne einfach zusammenhängend ist, deren Kurve K also nicht zu einer Schleife geschlossen ist. Die Maximalausdehnung der Schnittfläche R ergibt sich damit durch die Länge 13 der Kurve K. Wiederum wird der Fokus lateral entlang der Kurve K verstellt. Während des Abfahrens der Länge 13 wird der Fokus zugleich axial oszillierend verstellt.

Die absolute Lage der axialen oberen und unteren Fokusposition variiert dabei längs der Kurve K. Dies ist natürlich nicht zwingend, es können auch konstante axiale obere und untere Fokuspositionen verwendet werden. Genauso wenig muss der Abstand zwischen der axialen oberen und unteren Fokusposition, zwischen denen die Oszillation ausgeführt wird, konstant sein. Im Ergebnis folgt die Lage des Fokus der in der Schnittfläche 9 mäandernden Bahnkurve 10, und die axiale obere(n) bzw. untere(n) Fokusposition(en) der Oszillation gibt (geben) den oberen bzw. unteren Rand der Schnittfläche 9 vor. Dies ist gleichermaßen für zweifach zusammenhängende Schnittflächen möglich.

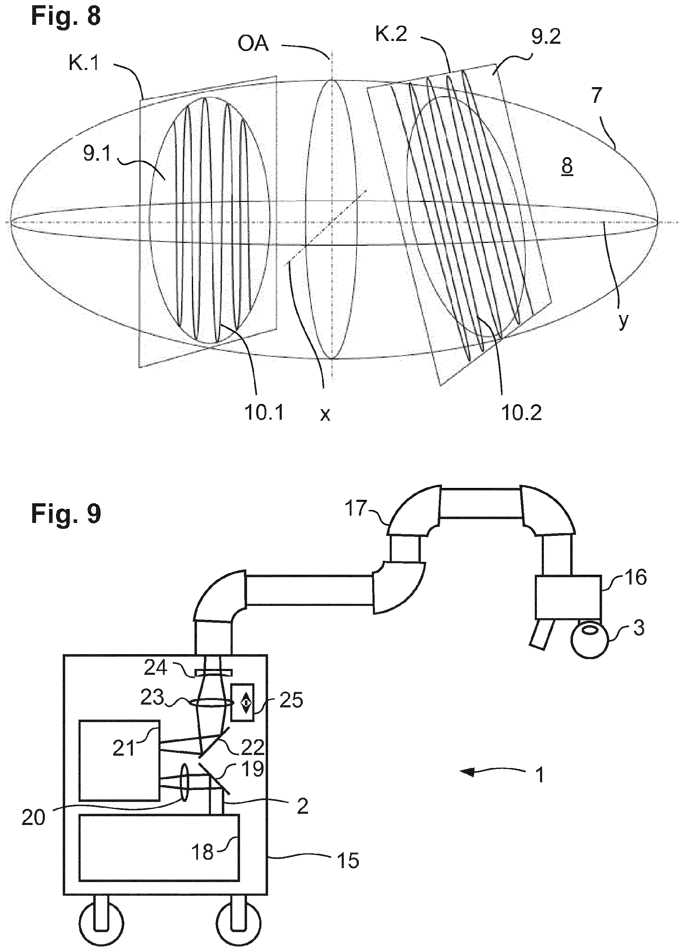

Fig. 8 zeigt exemplarisch mögliche Anwendungen für solche Schnittflächen. Es sind je zwei Schnittflächen wiederum an einem Kapselsack, umhüllend eine Augenlinse, dargestellt. Exemplarisch ist eine nicht unter die Ansprüche fallende Schnittfläche 9.1 und eine die Erfindung realisierende Schnittfläche 9.2 gezeigt, die Kurven K.1 bzw. K.2 haben.

Die linke Schnittfläche 9.1 ist ein Beispiel dafür, wie die obere und untere Fokusposition während des Verschiebens entlang der Kurve K.1 variieren kann. Die Oszillation entlang der Bahnkurve 10.1 ist somit zur Lage längs der Kurve K.1 synchronisiert, so dass z. B. die in Fig. 8 gezeigte kreisscheibenförmige Schnittfläche erzeugt wird. Damit ist es, wie im Ausführungsbeispiel der Fig. 8 gezeigt, möglich, die Schnittfläche auf einen gewünschten Abschnitt des transparenten Materials zu beschränken, hier auf die Augenlinse, ohne andere Materialstrukturen zu beeinträchtigen, hier den Kapselsack.

Die rechte Schnittfläche 9.2 sieht vor, dass die Erfindung von einer Parallelität der Schnittfläche zur optischen Achse OA auch abweicht. Die axiale Fokusverstellung wird mit der lateralen Fokusverstellung dahingehend synchronisiert, dass eine zusätzlich laterale Fokusverstellung synchronisiert zur axialen Fokusverstellung ausgeführt wird, um die Schnittfläche 9.2 leicht gegen die optische Achse OA zu neigen. Natürlich kann diese Neigung auch auf Abschnitte der Schnittfläche 9.2 beschränkt sein. Besonders einfach ist diese zusätzliche laterale Fokusverstellung beim Behandlungsgerät der Fig. 2 dann zu erreichen, wenn die zusätzlich Fokusverstellung im Bildfeld B geeignet aktiviert und betätigt wird. Die Schnittfläche 9.2 ist immer noch im Wesentlichen parallel zur optischen Achse OA, da die zusätzliche laterale Fokusverstellung in Synchronisation zur axialen Fokusverstellung klein gegen die Ausdehnung der Kurve K ist.

Die axiale Fokusverschiebung kann einfach ausgeführt werden, wenn eine Minimierung der Amplitude erfolgen kann. Bevorzugt kann dies erfolgen, indem ein z-Scanner so gestaltet wird, dass das optische Übersetzungsverhältnis von Fokusbewegung zu z-Scanner-Bewegung weniger als 1:2 beträgt, vorzugsweise sogar weniger als 1:1. Das heißt, dass die mechanische Wegvariation im Scanner nicht größer ist als die Fokusbewegung im Objekt. Dann liegt dann die Beschleunigung im Bereich 0,1 bis 10$^3$ m/s$^2$.

Ein optionales Mittel zur Erreichung eines solchen Wertes besteht darin, einen reflektiven z-Scanner zu verwenden, dessen optisches Design so beschaffen ist, dass vermieden ist, dass ein Strahlfokus bei einer üblichen Scan-Bahn auf eine optische Grenzfläche des Scanners positioniert wird.

Ein weiteres optionales Mittel besteht darin, dass der z-Scanner als Antrieb einen Piezo-Stack oder eine Tauchspule aufweist, die möglichst resonant betrieben werden. Auch eine Option ist es, die Reflektion mittels eines elektrooptischen Bauelements (z. B. AOM) zu bewirken.

Wie erwähnt, kann auch ein zweiter z-Scanner verwendet werden, der zusätzliche zeitlich langsame Divergenzänderungen (positiv oder negativ) des Laserstrahls realisiert, wobei die Ansteuerung der Scanner durch eine Steuereinheit erfolgt, die die Positionssignale (Messsignal) oder Steuersignale eines Scanners bei der Ansteuerung des jeweils anderen berücksichtigt, weil beispielsweise beide Steuersignale dort erzeugt werden. Die Steuereinheit kann durch die Steuereinrichtung 6 realisiert werden.

Für eine schnelle Variation der Fokuslage entlang der optischen Achse, d.h. für einen schnellen z-Scanner, im Bereich der ophthalmologischen Chirurgie, insbesondere für Katarakt-Operationen ist das in Fig. 9 schematisch dargestellte Behandlungsgerät 1 vorgesehen. In diesem Behandlungsgerät 1 sind Elemente, die bereits anhand der vorherigen Figuren erläutert wurde und die im Behandlungsgerät 1 funktions- oder strukturgleich sind, mit denselben Bezugszeichen versehen und werden deshalb nicht zwingend nochmals erläutert.

Das Behandlungsgerät 1 weist ein Basisteil 15 auf, welches die Laserstrahlung 2 bereitstellt und ein Handteil 16, an das die Laserstrahlung übertragen wird. Die Übertragung erfolgt mittels eines Gelenkarmes 17, der bevorzugt eine Freistrahloptik aufweist, die beispielsweise durch geeignete Umlenkspiegel (nicht dargestellt) realisiert sein kann. Die Verwendung von Lichtleitfasern ist nachteilig, da Standprobleme bezüglich der erforderlichen Strahlungsintensitäten der Laserstrahlung 2 bestehen. Lichtleitfasern sind im oder anstelle des Gelenkarms 17 dennoch möglich.

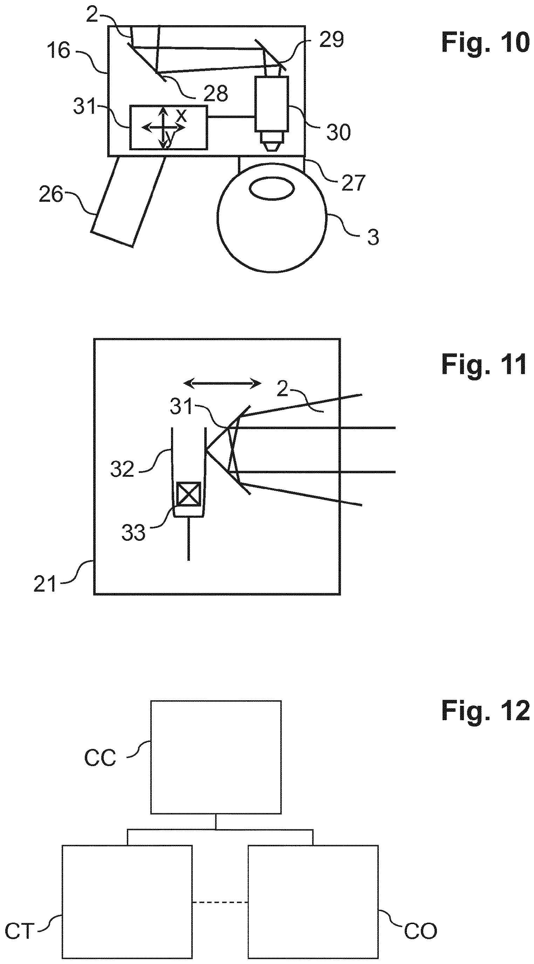

Das Handteil 16 wird auf das Auge 3 aufgesetzt. Es ist in Fig. 10 detailliert gezeigt und wird später noch erläutert werden.

Die z-Scanner und xy-Scanner sind im Basisteil 15 und im Handteil 16 realisiert. Beide Elemente bilden somit zusammen mit dem Gelenkarm 17 die Lasereinrichtung L. In der Bauweise der Fig. 9 ist der z-Scanner im Basisteil 15 vorgesehen. Eine Laserquelle 18 gibt die Laserstrahlung 2 ab, die über einen Umlenkspiegel 19 und eine Linse 20 in einem nicht-kollimierten Strahlengang in den z-Scanner 21 geleitet wird, dessen Aufbau später anhand der Figuren 11, 13, 14 noch erläutert werden wird. Aus dem z-Scanner gelangt die Laserstrahlung 2 über einen Umlenkspiegel 22 und Linsen 23, 24 als nicht-kollimiertes Bündel in den Gelenkarm 17. Die Linsen 23, 24 bilden ein Teleskop, das über einen Antrieb 25 verstellbar ist. Die Bedeutung dieses Teleskops wird noch erläutert werden.

Der Gelenkarm 17 beinhaltet bei Ausführung in Freistrahloptik eine Reihe von Umlenkspiegeln und gegebenenfalls eine Relayoptik, um das nicht-kollimierte Bündel zum Ausgang des Gelenkarms zu übertragen.

Das Handteil 16 empfängt die Laserstrahlung 2 als nicht-kollimiertes Bündel aus dem Gelenkarm 17 und gibt sie über ein Kontaktglas 27 in das Auge 3 ab. Zur Positionierung des Handteils 16 ist ein Griff 26 vorgesehen.

Die Laserstrahlung 2 wird im Handteil 16 über Umlenkspiegel 28 und 29 zu einem Objektiv 30 gebracht, das von einem Stellglied 31 quer zur optischen Achse verschoben wird. Dies realisiert die bereits erwähnte Bildfeldverstellung. Das Objektiv 30 ist kein Feldobjektiv, d. h. kein bildseitig telezentrisches Objektiv, vielmehr wird je nach Divergenz bzw. Konvergenz des Bündels an der Eingangspupille des Objektivs 30 die Laserstrahlung in unterschiedliche Tiefen im Auge 3 fokussiert.

Damit wird die vom z-Scanner bewirkte Variation der Bündelausbreitung in eine Variation der axialen Fokusposition im Auge 3 umgesetzt.

Zur Verstellung des Bildfeldes wird das Objektiv 3 im Handteil 16 mit Hilfe des Stellgliedes 31 lateral bewegt. Zugleich sind die Umlenkspiegel 28 und 29 im Handteil 16 mechanisch angesteuert und werden von der Steuereinrichtung C der Verschiebung des Objektivs 30 so nachgeführt, dass die Laserstrahlung 2 als Bündel immer in der Eintrittspupille des Objektivs zentriert bleibt.

Fig. 11 zeigt schematisch die Wirkung des z-Scanners 21. Er verfügt über einen Eckspiegel 31, der einen Retro-Reflektor realisiert. Grundsätzlich könnte der Eckspiegel 31 durch einen andersartigen Retro-Reflektor ersetzt werden. Der Eckspiegel 31 ist an einer Stimmgabel 32 befestigt, welche von einem Erreger 33 in Schwingungen versetzt wird. Der Eckspiegel ist mit dem nicht-kollimierten Bündel der Laserstrahlung 2 beleuchtet. Durch die Verstellung der Position des Eckspiegels wird die Länge des nicht-kollimierten Strahlengangs verändert und damit die Ausbreitung des divergierenden Bündels der Laserstrahlung 2.

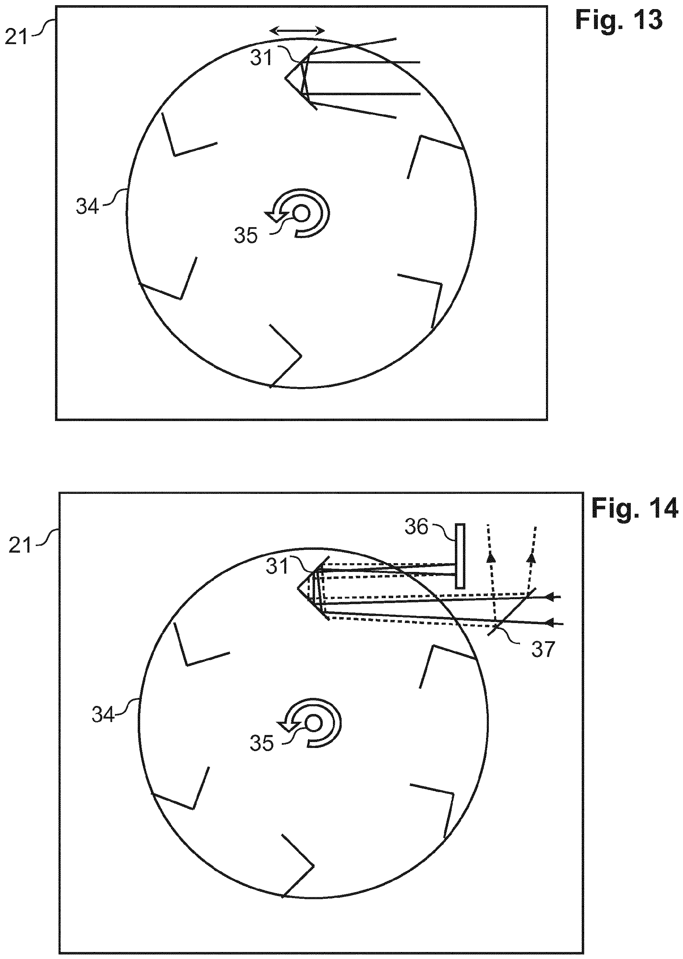

Die Fig. 13 und 14 zeigen Alternativen für den Scanner 21. Gemäß Fig. 13 sind eine Vielzahl von Eckspiegeln 31 auf einer rotierenden Scheibe 34 befestigt, die um eine Achse 35 in Rotation versetzt wird. Die Rotation bewirkt, dass die optische Weglänge des divergierenden Strahlenteils verstellt wird. Der Vorteil ist eine schnellere Verstellung, der Nachteil eine geringere Wiederholgenauigkeit und ein erhöhter Justieraufwand für die mehreren Eckspiegel 31. Fig. 14 zeigt eine nochmalige Weiterbildung, die zusätzlich zur Bauweise der Fig. 13 noch einen Endspiegel 36 sowie einen Strahlteiler 35 hat. Das einfallende divergierende Bündel der Laserstrahlung 2 durchläuft den Strahlteiler 37, wird am Eckspiegel 31 zum Endspiegel 36 umgelenkt, dort reflektiert (ab nun ist der Strahlengang gestrichelt gezeichnet), wiederum vom Eckspiegel 31 reflektiert und dann vom Strahlteiler 37 ausgekoppelt. Die Weglängenverstellung, die sich bei der Drehung der Scheibe 34 ergibt ist damit doppelt so groß, so dass bei gleicher Drehgeschwindigkeit der Scheibe 34 eine höhere Verstellgeschwindigkeit erreicht wird.

Um gezielte Schnitte im Auge gemäß der vorbeschriebenen Schnittführung durchzuführen, wird das Objektiv 31 im Handteil 16 vom Stellglied 31 (unter Nachführung der Umlenkspiegel 28 und 29) lateral bewegt. Während dieser lateralen Bewegung wird das Teleskop 23, 24 durch den Antrieb 25 synchron nachgestellt. Dies ist notwendig, weil durch die laterale Bewegung des Objektivs 31 die optische Weglänge vom Scanner 21 zum Objektiv 30 variiert wird. Da das Bündel der Laserstrahlung 2 auf das Objektiv 30 nicht-kollimiert einfällt, würde die Weglängenänderung zusätzlich zu einer Änderung der z-Position des Fokus führen. Die gewünschte z-Scannereigenschaft wäre damit gestört. Deshalb wird die Divergenz/Konvergenz des Lichtbündels mit Hilfe des Teleskops 23, 24 synchron zur Bewegung des Objektivs 30 verstellt.

Eine dafür vorgesehene steuerungstechnische Realisierung zeigt als Blockschaltbild die Fig. 12. Hier ist die Steuereinrichtung C in Form von drei Funktionen gezeigt. Ein zentraler Steuerblock CC steuert zwei Sub-Blöcke an, einen Steuerblock CT für die Ansteuerung des Teleskops 23, 24 und einen Steuerblock CO für das Stellglied 31, d.h. zur Verschiebung des Objektivs 30. Die steuerungstechnische Abstimmung der Verstellung der Querlage des Objektivs 30 und des Teleskops 23, 24 erreicht, dass die Lage des Fokus in Tiefenrichtung ausschließlich durch den z-Scanner 21 eingestellt wird, und dass die Verschiebung des Bildfeldes B keine gleichzeitige Verschiebung der Tiefenlage des Fokus mit sich bringt. Mit anderen Worten, die synchrone Verstellung von Teleskop 23, 24 als Mittel zum Beeinflussen der Divergenz/Konvergenz der auf das Objektiv 30 einfallenden Strahlung und die Querverschiebung des Objektivs sorgen dafür, dass die Verschiebung des Bildfeldes in einer Ebene senkrecht zur optischen Achse und nicht auf einer gekrümmten Bahn erfolgt.

Bei der Katarakt-Behandlung ist es vorteilhaft, Navigationseigenschaften bereitzustellen, welche die Lage der zu bearbeitenden Strukturen, beispielsweise die Lage des Kapselsacks oder der Linse aufzufinden erlaubt. Bei einem System mit einer mechanisch bewegten Optik zur lateralen Fokusverschiebung, die ein Bildfeld hat, welches kleiner ist als die zu findende Struktur oder die zu erzeugende Schnittfläche, kommen verschiedene Varianten in Frage.

Variante 1 - Konfokale Detektion: Zur Vermessung der Topographie des Auges 3 im Bereich der Hornhaut 5 und/oder der Linse 8 wird rückreflektiertes Licht mit Hilfe von Polarisationsoptiken umgeleitet, auf einer Blende fokussiert und mit Hilfe eines Photodetektors erfasst. Für diese Messung wird die Wellenlänge der Laserstrahlung 2 verwendet. Mit Hilfe des z-Scanners werden konfokale Signale von einem kleinen Scanbereich 10-100 $\mu$m erfasst. Die vom Photodetektor erfassten Signale werden verstärkt, z. B. mit einem Lock-In-Verfahren oder Boxcar Integrator. Die Referenzfrequenz für den Lock-in-Verstärker stimmt mit der Scanfrequenz des schnellen z-Scanners überein. Die konfokalen Signale werden während der (langsamen) Bewegung des Objektivs erfasst. Das Bildfeld B bewegt sich entlang der Kurve K, welches mit dem Muster für die Laserbehandlung übereinstimmt. Gleichzeitig wird die axiale Fokuslage verstellt, so dass eine Bahnkurve (z. B. die Bahnkurve 10) abgefahren wird. In dieser Art und Weise wird die genaue Position ermittelt, wo eine Laserdesorption erfolgen soll, und damit wird die Sicherheit der Behandlung erhöht.

Variante 2 - OCT Detektion: Bei dieser Variante wird die Topographie des Auges 3 im Bereich der Hornhaut 5 und/oder der Linse 8 mit kurzkohärentem Licht vermessen, welches eine andere Wellenlänge aufweist. Das Licht einer kurzkohärenten Quelle wird mit Hilfe eines dichroitischen Spiegels in die Optik O eingekoppelt. Das von Augenstrukturen rückreflektierte Licht wird durch denselben dichroitischen Spiegel umgeleitet und mit Hilfe einer interferometrischen Anordnung detektiert. Um die Sicherheit und Präzision der Laserbehandlung zu erzielen, wird ein Objektiv eingesetzt, welches eine numerische Apertur im Bereich 0,15-0,2 aufweist. Bei einer vollausgeleuchteten Eintrittspupille des Objektivs ist der z-axiale Messbereich der OCT-Detektion durch die Schärfetiefe des Objektivs eingeschränkt. Demgemäß wird hierbei die kurzkohärente Beleuchtung so ausgelegt (z. B. durch entsprechende Auswahl der Kollimatorgeometrie oder mit Hilfe einer Blende), dass nur ein Teil der Eintrittspupille des Objektiv O ausgeleuchtet wird. Da die effektive numerische Apertur des fokussierten kurzkohärenten Lichtes dadurch verringert wird, wird hierbei ein größerer Tiefenbereich für die OCT-Detektion realisiert.

Es erfolgt bei diesen Navigationsmessungen bevorzugt keine tomographische Bildaufnahme der Augenstrukturen, sondern anhand tiefenaufgelöster Messungen an ausgewählte Stellen wird die Augenstruktur simuliert und an ein rechnerisches Augenmodell angepasst. Das Ergebnis dieser Anpassung wird als optionale Animation dargestellt. Anhand dieser Animation wird der Chirurg in der Lage versetzt, die räumlichen Grenzen der Photodesorption einzustellen.

Vorzugsweise erfolgen die tiefenaufgelösten Navigationsmessungen entlang der Bahnkurve, welche für die Laserbehandlung eingesetzt wird. Wenn anhand dieser Messungen eine Dezentrierung des kreisförmigen Musters der geplanten Kapselsacköffnung in Bezug auf die Augenlinse festgestellt wird, kann ggf. eine neue Navigationsmessreihe erfolgen. Die hiermit gewonnenen Daten werden mit dem rechnerischen Augenmodell erneut korreliert und das Ergebnis wird als Animation zur Kontrolle der Laserbehandlung angezeigt.

**Patentansprüche**

1. Bearbeitungsvorrichtung zur Erzeugung einer Schnittfläche in einem transparenten Material (7, 8), die umfasst

   - eine Lasereinrichtung (L), die dazu ausgebildet ist, mittels optischer Strahlung (2) eine Trennung innerhalb des transparenten Materials (7, 8) zu bewirken, und die aufweist

      - eine Optik (O), die optische Strahlung längs einer optischen Achse (OA) in einen im Material gelegenen Fokus (6) bündelt und im Material (7, 8) ein Bildfeld hat (B), in dem der Fokus (6) liegt und das eine Bildfeldgröße hat,
      - eine Einrichtung zur Verstellung einer Lage des Fokus (6) quer zur optischen Achse (OA) und längs der optischen Achse (OA),

      - eine Steuereinrichtung (S), welche mit der Lasereinrichtung (L) verbunden ist und die Lasereinrichtung (L) so ansteuert, dass deren Einrichtung zur Verstellung der Lage des Fokus (6) die Lage des Fokus (6) der optischen Strahlung (2) im Material (7, 8) entlang einer Bahnkurve (10.2) verschiebt, wobei die Steuereinrichtung (S) die Lasereinrichtung (L) so ansteuert, dass die Schnittfläche (9.2) senkrecht zur optischen Achse (OA) eine Kurve (K.2) hat, wobei die Steuereinrichtung (S) für die Einrichtung zur Verstellung der Lage des Fokus (6) quer zur optischen Achse (OA) eine laterale Bewegung der Lage des Fokus (6), die in der Schnittebene entlang der Kurve (K.2) läuft, vorgibt und längs der optischen Achse (OA) während der lateralen Bewegung eine mehrfach axial oszillierende, axiale Verstellung der Lage des Fokus (6) zwischen einer oberen axialen Fokusposition und einer unteren axialen Fokusposition vorgibt, wobei optional obere axiale und untere axiale Fokuspositionen entlang der Kurve (K.2) variieren und/oder optional ein Abstand zwischen oberer axialer und unterer axialer Fokusposition entlang der Kurve (K.2) nicht konstant ist, **dadurch gekennzeichnet, dass** die Steuereinrichtung (S) die laterale Fokusverstellung mit der axialen Fokusverstellung synchronisiert und mindestens abschnittsweise eine zusätzliche laterale Fokusverstellung synchronisiert zur axialen Fokusverstellung vorgibt, so dass die Schnittfläche (9.2) mindestens abschnittsweise gegenüber der optischen Achse (OA) geneigt ist, wobei die zusätzliche laterale Fokusverstellung klein gegen die Ausdehnung der Kurve (K.2) ist.

2. Vorrichtung nach Anspruch 1, **dadurch gekennzeichnet, dass** die Steuereinrichtung (S) auf Abschnitten (11) der Bahnkurve, in den sich die Lage des Fokus (6) mit einer Einfallsrichtung der optischen Strahlung (2) bewegt, die optische Strahlung (2) abschaltet oder so modifiziert, dass die optische Strahlung (2) keine Materialtrennungswirkung im transparenten Material (7, 8) hat.

3. Vorrichtung nach Anspruch 2, **dadurch gekennzeichnet, dass** die Oszillation asymmetrisch ist, wobei die Abschnitte (11) der Bahnkurve, in den sich die Lage des Fokus (6) mit der Einfallsrichtung der optischen Strahlung (2) bewegt, steiler verlaufen, als Abschnitte (12) der Bahnkurve, in den sich die Lage des Fokus (6) entgegen der Einfallsrichtung der optischen Strahlung (2) bewegt.

4. Vorrichtung nach einem der obigen Ansprüche, **dadurch gekennzeichnet, dass** die Lasereinrichtung (L) ein Basisteil (15), ein bewegliches Handteil (16) und eine flexible oder gelenkige, optische Übertragungseinrichtung (17) aufweist, welche das Basisteil (15) und das Handteil (16) verbindet, wobei das Basisteil (15) die Laserstrahlung (2) in die Übertragungseinrichtung (17) als nicht-kollimiertes Bündel einkoppelt, die Übertragungseinrichtung (17) das nicht-kollimierte Bündel zum Handteil (16) leitet, das Handteil (16) ein Objektiv (30) aufweist, welches das nicht-kollimierte Bündel aufnimmt und in das Auge (3) fokussiert, und die Einrichtung zur Verstellung der Lage des Fokus (6) zweiteilig ausgebildet ist und einen ersten Scanner (21), der die Lage des Fokus (6) längs der optischen Achse (OA) verstellt und im Basisteil (15) angeordnet ist, und einen zweiten Scanner (31, 23, 24, 25) aufweist, der die Lage des Fokus (6) quer zur optischen Achse (OA) verstellt, wobei der zweite Scanner ein Stellglied (31) zur Verschiebung des Objektivs (30) quer zur optischen Achse (OA) und eine Divergenzverstelleinrichtung (23-25) umfasst, welche eine Divergenz oder Konvergenz des nicht-kollimierten Bündels beeinflusst, und wobei weiter die Steuereinrichtung so ausgebildet ist, dass sie die die Divergenzverstelleinrichtung (23-25) synchron mit dem Stellglied (31) zur Verschiebung des Objektivs (30) ansteuert.

5. Vorrichtung nach Anspruch 4, **dadurch gekennzeichnet, dass** die Divergenzverstelleinrichtung (23-25) im Basisteil (15) angeordnet ist.

6. Vorrichtung nach Anspruch 4 oder 5, **dadurch gekennzeichnet, dass** die Divergenzverstelleinrichtung ein Teleskop (23, 24) und einen dieses verstellenden Aktuator (25) aufweist.

7.  Vorrichtung nach Anspruch 4, 5 oder 6, **dadurch gekennzeichnet, dass** der erste Scanner (21) zur Verstellung der Lage des Fokus (6) längs der optischen Achse (OA) eine Weglänge des nicht-kollimierten Bündels verändert, bevorzugt mittels mindestens einen verschieblichen Eckspiegels (31), wobei optional der erste Scanner (21) einen Schwinger (32) zur periodischen Veränderung der Weglänge oder mehrere Eckspiegel (31) aufweist, die auf einer rotierenden Scheibe (34) montiert sind.

8.  Vorrichtung nach einem der Ansprüche 4 bis 7, **dadurch gekennzeichnet, dass** das Handstück eine Nachführeinrichtung (28, 29) aufweist, die synchron zur Verschiebung des Objektivs (30) das nicht-kollimierte Bündel in einer Pupille des Objektivs (30) zentriert hält.

9.  Verfahren zur Erzeugung von Steuerdaten für eine Erzeugung einer Schnittfläche in einem transparenten Material (7, 8), wobei

    - die Steuerdaten so erzeugt werden, dass sie für eine Lasereinrichtung (L) ausgebildet sind, die mittels optischer Strahlung (2) eine Trennung innerhalb des transparenten Materials (7, 8) bewirkt, und die aufweist

        - eine Optik (O), die optische Strahlung (2) längs einer optischen Achse (OA) in einen im Material gelegenen Fokus (6) bündelt und im Material (7, 8) ein Bildfeld (B) hat, in dem der Fokus (6) liegt und das eine Bildfeldgröße hat,
        - eine Einrichtung zur Verstellung einer Lage des Fokus (6) quer zur optischen Achse (OA) und längs der optischen Achse (OA),

    - die Steuerdaten eine Bahnkurve (10.2) für eine Verstellung der Lage des Fokus (6) der optischen Strahlung (2) im Material (7, 8) zur Erzeugung der Schnittfläche (9.2) derart vorgeben, dass die Schnittfläche (9.2) senkrecht zur optischen Achse (OA) eine Kurve (K.2) hat, wobei die Steuerdaten so erzeugt werden, dass sie zur Bewegung des Fokus auf der Bahnkurve (10) für die Einrichtung zur Verstellung der Lage des Fokus (6) eine laterale Bewegung der Lage des Fokus (6), die in der Schnittebene entlang der Kurve (K.2) läuft, vorgeben und längs der optischen Achse (OA) während der lateralen Bewegung eine mehrfach axial oszillierende, axiale Verstellung die Lage des Fokus (6) zwischen einer oberen axialen Fokusposition und einer unteren axialen Fokusposition vorgeben, wobei optional obere axiale und untere axiale Fokuspositionen entlang der Kurve (K.2) variieren und/oder optional ein Abstand zwischen oberer axialer und unterer axialer Fokusposition entlang der Kurve (K.2) nicht konstant ist, **dadurch gekennzeichnet, dass** die Steuerdaten so erzeugt werden, dass sie die laterale Fokusverstellung mit der axialen Fokusverstellung synchronisieren und mindestens abschnittsweise eine zusätzliche laterale Fokusverstellung synchronisiert zur axialen Fokusverstellung vorgeben, so dass die Schnittfläche (9.2) mindestens abschnittsweise gegenüber der optischen Achse (OA) geneigt ist, wobei die zusätzliche laterale Fokusverstellung klein gegen die Ausdehnung der Kurve (K.2) ist.

10. Verfahren nach Anspruch 9, **dadurch gekennzeichnet, dass** die Steuerdaten auf Abschnitten (11) der Bahnkurve, in den sich die Lage des Fokus (6) mit einer Einfallsrichtung der optischen Strahlung (2) bewegt, die optische Strahlung (2) abschalten oder so modifizieren, dass die optische Strahlung (2) keine Materialtrennungswirkung im transparenten Material (7, 8) hat.

11. Verfahren nach Anspruch 10, **dadurch gekennzeichnet, dass** die Oszillation asymmetrisch ist, wobei die Abschnitte (11) der Bahnkurve, in den sich die Lage des Fokus (6) mit der Einfallsrichtung der optischen Strahlung (2) bewegt, steiler verlaufen, als Abschnitte (12) der Bahnkurve, in den sich die Lage des Fokus (6) entgegen der Einfallsrichtung der optischen Strahlung (2) bewegt.

12. Verfahren oder Vorrichtung nach einem der obigen Ansprüche, **dadurch gekennzeichnet, dass** axial oszillierende, axiale Verstellung der Lage des Fokus (6) zwischen einer oberen axialen Fokusposition (z1), die einen oberen Rand der Schnittfläche (10) definiert, und einer unteren axialen Fokusposition (z2), die einen unteren Rand der Schnittfläche (10) definiert, verläuft, wobei die oberen und/oder untere axiale Fokusposition längs der Kurve (K.2) variiert.

13. Verfahren oder Vorrichtung nach einem der obigen Ansprüche, **dadurch gekennzeichnet, dass** zur Verstellung der Lage des Fokus (6) quer zur optischen Achse (OA) eine Verschiebung des Bildfeldes (B) bewirkt ist, wobei die zusätzliche laterale Fokusverstellung eine Verstellung der Lage des Fokus (6) im Bildfeld (B) ist.

14. Verfahren oder Vorrichtung nach einem der obigen Ansprüche, **dadurch gekennzeichnet, dass** die Kurve eine periodische Lissajous-Figur ist.

**15.** Verfahren oder Vorrichtung nach einem der obigen Ansprüche, **dadurch gekennzeichnet, dass** die Kurve eine geschlossene Kurve ist.

**Claims**

**1.** A treatment apparatus for producing a cut in a transparent material (7, 8), which comprises

- a laser device (L) which is configured to generate a cut within the transparent material (7, 8) by means of optical radiation (2) and which laser device comprises

- optics (O) which focus the optical radiation along an optical axis (OA) into a focus (6) situated in the material and which have in the material (7, 8) an image field (B) in which the focus (6) lies and which has an image field size, and
- a focus (6) position adjustment device for moving a position of the focus (6) transverse to the optical axis (OA) and along the optical axis (OA),

- a control device (S) which is connected to the laser device (L) and controls the laser device (L) such that the focus (6) position adjustment device moves the position of the focus (6) of the optical radiation (2) in the material (7, 8) along a path (10.2), wherein the control device (S) controls the laser device (L) such that, perpendicular to the optical axis (OA), comprises the cut (9.2), wherein the control device (S) prescribes for the focus (6) position adjustment device a lateral movement of the positon of the focus (6) transvers to the optical axis (OA) which is located in a sectional plane along the curve (K.2) and prescribes along the optical axis (OA) during the lateral movement a multiple axially oscillating axial adjustment of the position of the focus (6) between an upper axial focus position and a lower axial focus position, wherein optionally the upper axial and the lower axial focus position vary along the curve (K.2) and/or optionally a distance between the upper axial and the lower axial focus position are not constant along the curve (K.2), **characterized in that** the control device (S) synchronizes the lateral focus adjustment to the axial focus adjustment and prescribes at least in sections an additional lateral focus adjustment synchronous to the axial focus adjustment such that the cut (9.2) is at least in sections inclined against the optical axis (OA), wherein the additional lateral focus adjustment is small compared to the extent of the curve (K.2).

**2.** The apparatus according to claim 1, **characterized in that** the control device (S) switches off or modifies the optical radiation (2) when the focus is on sections (11) of the path (10) in which the position of the focus (6) moves with a direction of incidence of the optical radiation (2), such that the optical radiation (2) has no material-cutting effect in the transparent material (7, 8) on such sections.

**3.** The apparatus according to claim 2, **characterized in that** the oscillation movement is asymmetrical, wherein the sections (11) of the trajectory (10) in which the position of the focus (6) moves with the direction of incidence of the optical radiation (2) run more steeply than other sections (12) of the path in which the position of the focus (6) moves contrary to the direction of incidence of the optical radiation (2).

**4.** Apparatus according to one of the above claims, **characterized in that** the laser device (L) comprises a base part (15), a movable handle (16) and a flexible or articulated, optical transmission device (17), which connects the base part (15) and the handle (16), wherein the base part (15) couples the laser radiation (2) into the transmission device (17) as a non-collimated beam, the transmission device (17) transmits the non-collimated beam to the handle (16), the handle (16) comprises an objective lens (30), which receives the non-collimated beam and focuses it into the eye (3), and the focus adjustment device for moving the position of the focus (6) is formed by two parts and has a first scanner (21), which moves the position of the focus (6) along the optical axis (OA) and is arranged in the base part (15), and a second scanner (31, 23, 24, 25), which moves the position of the focus (6) transverse to the optical axis (OA), wherein the second scanner comprises an actuator (31) for displacing the objective lens (30) transverse to the optical axis (OA) and a divergence-modifying device (23-25), which modifies a divergence or convergence of the non-collimated beam, and wherein furthermore the control device is configured such that it controls the divergence-modifying device (23-25) to act synchronously with the actuator (31) for displacing the objective lens (30).

**5.** The apparatus according to claim 4, **characterized in that** the divergence-modifying device (23-25) is arranged in the base part (15).

**6.** The apparatus according to claim 4 or 5, **characterized in that** the divergence-modifying device comprises a telescope (23, 24) and an actuator (25) that adjusts it.

**7.** The apparatus according to claim 4, 5 or 6, **characterized in that** for moving the position of the focus (6) along the optical axis (OA) the first scanner (21) changes a path length of the non-collimated beam, preferably by means of at least one displaceable corner mirror (31), wherein optionally the first scanner (21) comprises an oscillator (32) for periodically changing the path length or several corner mirrors (31), which are mounted on a rotating disc (34).

**8.** The apparatus according to one of claims 4 to 7, **characterized in that** the handpiece comprises a tracking device (28, 29) which holds the non-collimated beam centred in a pupil of the objective lens (30) synchronously with the displacement of the objective lens (30).

**9.** A method for producing control data for producing a cut in a transparent material (7, 8), wherein

- the control data are configured for a laser device (L) which is configured to generate a cut within the transparent material (7, 8) by means of optical radiation (2) and which laser device comprises

- optics (O) which focus the optical radiation (2) along an optical axis (OA) into a focus (6) situated in the material and which have in the material (7, 8) an image field (B) in which the focus (6) lies and which has an image field size,
- a focus (6) position adjustment device for moving a position of the focus (6) transverse to the optical axis (OA) and along the optical axis (OA),

- wherein the control data define a path (10.2) for moving the position of the focus (6) of the optical radiation (2) in the material (7, 8) to produce the cut (9.2) in such a way that the cut (9.2) comprises a curve (K2) perpendicular to the optical axis (OA), wherein the control data are generated such that they prescribe to adjust of the focus on the path (10), for the focus (6) position adjustment device a lateral movement of the position of the focus (6) running in the sectional plane along the curve (K.2) and, during the lateral movement, a multiple axially oscillating axial adjustment of the position of the focus (6) along the optical axis (OA) between an upper axial focus position and a lower axial focus position, wherein optionally the upper axial and the lower axial focus positons vary along the curve (K.2) and/or optionally a distance between the upper axial and the lower axial focus positions is not constant along the curve (K.2), **characterized in that** the control data are generated such that they synchronize the lateral focus adjustment to the axial focus adjustment and prescribe at least in sections an additional lateral focus adjustment synchronized to the axial focus adjustment such that the cut (9.2) is at least in sections inclined against the optical axis (OA), wherein the additional lateral focus adjustment is small against the extent of the curve (K.2).

**10.** The method according to claim 9, **characterized in that** on sections (11) of the path in which the position of the focus (6) moves with a direction of incidence of the optical radiation (2), the optical radiation (2) is switched off or modified such that the optical radiation (2) has no material-cutting effect in the transparent material (7, 8) on such sections.

**11.** The method according to claim 10, **characterized in that** the oscillating movement is asymmetrical, wherein the sections (11) of the trajectory in which the position of the focus (6) moves with the direction of incidence of the optical radiation (2) run more steeply than other sections (12) of the path (10) in which the position of the focus (6) moves contrary to the direction of incidence of the optical radiation (2).

**12.** The apparatus or the method according to any of the above claims, **characterized in that** the axially oscillating axial adjustment of the position of the focus (6) runs between an upper axial focus position (z1) defining an upper border of the cut (10) and a lower axial focus position (z2) defining a lower border of the cut (10), wherein the upper and/or the lower axial focus positions vary along the curve (K.2).

**13.** The apparatus or the method according to any of the above claims, **characterized in that** a shift of the image field (B) is effected to adjust the position of the focus (6) perpendicular to the optical axis (OA), wherein the additional lateral focus adjustment is an adjustment of the position of the focus (6) within the image field (B).

**14.** The apparatus or the method according to one of the above claims, **characterized in that** the curve is a periodic Lissajous figure.

**15.** The apparatus or the method according to one of the above claims, **characterized in that** the curve is a closed curve.

**Revendications**

**1.** Dispositif de traitement destiné à produire une surface de coupe dans une matière transparente (7, 8), ledit dispositif comprenant

- une unité laser (L) qui est conçue pour provoquer une séparation à l'intérieur de la matière transparente (7, 8) au moyen d'un rayonnement optique (2), et qui comporte

- une optique (O) qui focalise le rayonnement optique le long d'un axe optique (OA) en un foyer (6) situé dans la matière et qui possède dans la matière (7, 8) un champ image (B) dans lequel est situé le foyer (6) et qui a une dimension,
- une unité de réglage d'une position du foyer (6) transversalement à l'axe optique (OA) et longitudinalement à l'axe optique (OA),

- une unité de commande (S) qui est reliée à l'unité laser (L) et qui commande l'unité laser (L) de telle sorte que son unité de réglage de la position du foyer (6) décale la position du foyer (6) du rayonnement optique (2) dans la matière (7, 8) le long d'une trajectoire (10.2), l'unité de commande (S) commandant l'unité laser (L) de sorte que la surface de coupe (9.2) présente une courbe (K.2) perpendiculairement à l'axe optique (OA), l'unité de commande (S) destinée à l'unité de réglage de la position du foyer (6) transversalement à l'axe optique (OA) spécifiant un déplacement latéral de la position du foyer (6) qui s'étend dans le plan de coupe le long de la courbe (K.2) et spécifiant, longitudinalement à l'axe optique (OA) pendant le déplacement latéral, un réglage axial, à oscillation axiale multiple, de la position du foyer (6) entre une position de foyer axiale supérieure et une position de foyer axiale inférieure, éventuellement les positions de foyer axiale supérieure et axiale inférieure variant le long de la courbe (K.2) et/ou éventuellement une distance entre la position de foyer axiale supérieure et la position de foyer axiale inférieure n'étant pas constante le long de la courbe (K.2), **caractérisé en ce que** l'unité de commande (S) synchronise le réglage de foyer latéral avec le réglage de foyer axial et spécifie au moins par portions un réglage de foyer latéral supplémentaire de manière synchronisée avec le réglage de foyer axial de sorte que la surface de coupe (9.2) soit inclinée au moins par portions par rapport à l'axe optique (OA), le réglage de foyer latéral supplémentaire étant faible par rapport à l'extension de la courbe (K.2).

**2.** Dispositif selon la revendication 1, **caractérisé en ce que**, sur des portions (11) de la trajectoire dans lesquelles la position du foyer (6) se déplace avec une direction d'incidence du rayonnement optique (2), l'unité de commande (S) désactive le rayonnement optique (2) ou le modifie de sorte que le rayonnement optique (2) n'ait pas d'effet séparateur de matière dans la matière transparente (7, 8).

**3.** Dispositif selon la revendication 2, **caractérisé en ce que** l'oscillation est dissymétrique, les portions (11) de la trajectoire, dans lesquelles la position du foyer (6) se déplace avec la direction d'incidence du rayonnement optique (2), étant plus inclinées que les portions (12) de la trajectoire dans lesquelles la position du foyer (6) se déplace dans la direction opposée à la direction d'incidence du rayonnement optique (2).

**4.** Dispositif selon l'une des revendications précédentes, **caractérisé en ce que** l'unité laser (L) comporte une base (15), une poignée mobile (16) et une unité de transmission optique flexible ou articulée (17) qui relie la base (15) et la poignée (16), la base (15) injectant par couplage le rayonnement laser (2) dans l'unité de transmission (17) sous la forme d'un faisceau non collimaté, l'unité de transmission (17) guidant le faisceau non collimaté vers la poignée (16), la poignée (16) comportant un objectif (30) qui reçoit le faisceau non collimaté et le focalise dans l'œil (3), et l'unité de réglage de la position du foyer (6) étant conçue en deux parties et comportant un premier scanner (21), qui règle la position du foyer (6) le long de l'axe optique (OA) et est disposé dans la base (15), et un deuxième scanner (31, 23, 24, 25) qui règle la position du foyer (6) transversalement à l'axe optique (OA), le deuxième scanner comprenant un actionneur (31) destiné à décaler l'objectif (30) transversalement à l'axe optique (OA) et une unité de réglage de divergence (23-25) qui influe sur une divergence ou convergence du faisceau non collimaté, et l'unité de commande étant conçue de façon à activer l'unité de réglage de divergence (23-25) de manière synchrone avec l'actionneur (31) afin de décaler l'objectif (30).

**5.** Dispositif selon la revendication 4, **caractérisé en ce que** l'unité de réglage de divergence (23-25) est disposée dans la base (15).

**6.** Dispositif selon la revendication 4 ou 5, **caractérisé en ce que** le dispositif de réglage de divergence comporte une lunette (23, 24) et un actionneur (25) qui règle celle-ci.

**7.** Dispositif selon la revendication 4, 5 ou 6, **caractérisé en ce que** le premier scanner (21) destiné à régler la position du foyer (6) longitudinalement à l'axe optique (OA) modifie une longueur de trajet du faisceau non collimaté, de préférence au moyen d'au moins un miroir d'angle mobile (31), éventuellement le premier scanner (21) comprenant un oscillateur (32) destiné à modifier périodiquement la longueur de trajet ou une pluralité de miroirs d'angle (31) qui sont montés sur un disque rotatif (34).

**8.** Dispositif selon l'une des revendications 4 à 7, **caractérisé en ce que** la poignée comporte une unité d'asservissement (28, 29) qui maintient le faisceau non collimaté de manière centrée dans une pupille de l'objectif (30) de manière synchrone avec le décalage de l'objectif (30).

**9.** Procédé de génération de données de commande pour produire une surface de coupe dans une matière transparente (7, 8),

- les données de commande étant générées de manière à être conçues pour une unité laser (L) qui provoque une séparation à l'intérieur de la matière transparente (7, 8) au moyen d'un rayonnement optique (2), et qui comporte

- une optique (O) qui focalise le rayonnement optique (2) le long d'un axe optique (OA) en un foyer (6) situé dans la matière et qui possède dans la matière (7, 8) un champ image (B) dans lequel est situé le foyer (6) et qui a une dimension,
- une unité de réglage d'une position du foyer (6) transversalement à l'axe optique (OA) et longitudinalement à l'axe optique (OA),

- les données de commande spécifiant une trajectoire (10.2) destinée au réglage de la position du foyer (6) du rayonnement optique (2) dans la matière (7, 8) afin de produire une surface de coupe (9.2) de sorte que la surface de coupe (9.2) présente une courbe (K.2) perpendiculairement à l'axe optique (OA), les données de commande étant générées de manière à spécifier un déplacement latéral de la position du foyer (6) qui est effectué dans le plan de coupe le long de la courbe (K.2) afin de déplacer le foyer sur la trajectoire (10) destinée à l'unité de réglage de la position du foyer (6) et spécifiant, longitudinalement à l'axe optique (OA) pendant le déplacement latéral, un réglage axial, à oscillation axiale multiple, de la position du foyer (6) entre une position de foyer axiale supérieure et une position de foyer axiale inférieure, éventuellement les positions de foyer axiale supérieure et axiale inférieure variant le long de la courbe (K.2) et/ou éventuellement une distance entre la position de foyer axiale supérieure et la position de foyer axiale inférieure n'étant pas constante le long de la courbe (K.2), **caractérisé en ce que** les données de commande sont générées de façon à synchroniser le réglage de foyer latéral avec le réglage de foyer axial et spécifier au moins par portions un réglage de foyer latéral supplémentaire de manière synchronisée avec le réglage de foyer axial de sorte que la surface de coupe (9.2) soit inclinée au moins par portions par rapport à l'axe optique (OA), le réglage de foyer latéral supplémentaire étant faible par rapport à l'extension de la courbe (K.2).

**10.** Procédé selon la revendication 9, **caractérisé en ce que**, sur des portions (11) de la trajectoire dans lesquelles la position du foyer (6) se déplace avec une direction d'incidence du rayonnement optique (2), les données de commande désactivent le rayonnement optique (2) ou le modifient de sorte que le rayonnement optique (2) n'ait aucun effet de séparation de matière dans la matière transparente (7, 8).

**11.** Procédé selon la revendication 10, **caractérisé en ce que** l'oscillation est dissymétrique, les portions (11) de la trajectoire, dans lesquelles la position du foyer (6) se déplace avec la direction d'incidence du rayonnement optique (2), étant plus inclinées que les portions (12) de la trajectoire dans lesquelles la position du foyer (6) se déplace dans la direction opposée à la direction d'incidence du rayonnement optique (2).

**12.** Procédé ou dispositif selon l'une des revendications précédentes, **caractérisé en ce que** le déplacement axial, à oscillation axiale, de la position du foyer (6) est effectué entre une position de foyer axiale supérieure (z1), qui définit un bord supérieur de la surface de coupe (10), et une position de foyer axiale inférieure (z2), qui définit un bord inférieur de la surface de coupe (10), la position de foyer axiale supérieure et/ou inférieure variant le long de la courbe (K.2).

**13.** Procédé ou dispositif selon l'une des revendications précédentes, **caractérisé en ce qu'**un décalage du champ image (B) est effectué pour régler la position du foyer (6) transversalement à l'axe optique (OA), le réglage de foyer latéral supplémentaire étant un réglage de la position du foyer (6) dans le champ d'image (B).

**14.** Procédé ou dispositif selon l'une des revendications précédentes, **caractérisé en ce que** la courbe est une figure de Lissajous périodique.

**15.** Procédé ou dispositif selon l'une des revendications précédentes, **caractérisé en ce que** la courbe est une courbe fermée.

**Fig. 1**

**Fig. 3**

**Fig. 2**

## Fig. 4

## Fig. 5

## Fig. 6

## Fig. 7

**Fig. 8**

K.1   OA   K.2   9.2

9.1   7

8

y

10.1   x   10.2

**Fig. 9**

17

16

3

24

23   25

21   22

19

20   2

18   15

1

**Fig. 10**

**Fig. 11**

**Fig. 12**

**Fig. 13**

**Fig. 14**

**IN DER BESCHREIBUNG AUFGEFÜHRTE DOKUMENTE**

**In der Beschreibung aufgeführte Patentdokumente**

- US 20110022036 A1
- WO 2006074469 A
- US 2009171329 A
- EP 1486185 A
- US 7486409 B
- US 6590670 B

- DE 10034251
- EP 1159986 A2
- US 5549632 A
- DE 69500997 T2
- WO 2004032810 A2